(19) Europäisches Patentamt
European Patent Office
Office européen des brevets

(11) EP 0 815 927 A2

(12) EUROPÄISCHE PATENTANMELDUNG

(43) Veröffentlichungstag:
07.01.1998 Patentblatt 1998/02

(51) Int. Cl.⁶: **B01D 61/14**, B01D 65/08,
B01D 63/16, B01D 15/08,
C12M 3/06, B01D 29/01,
C07K 1/14

(21) Anmeldenummer: 97110650.5

(22) Anmeldetag: 30.06.1997

(84) Benannte Vertragsstaaten:
AT BE CH DE DK ES FI FR GB GR IE IT LI LU MC
NL PT SE

(30) Priorität: 28.06.1996 DE 29611336 U

(71) Anmelder:
Gesellschaft für Biotechnologische
Forschung mbH (GBF)
D-38124 Braunschweig (DE)

(72) Erfinder:
• Kroner, Karl-Heinz
38124 Braunschweig (DE)
• Vogel, Jens
38124 Braunschweig (DE)

(74) Vertreter:
Boeters, Hans Dietrich, Dr. et al
Patentanwälte Boeters & Bauer,
Bereiteranger 15
81541 München (DE)

(54) **Vorrichtung zur integrierten Zellabtrennung und Produktreinigung**

(57) Eine Filtrationsvorrichtung zur Abtrennung und/oder Anreicherung von Gelöststoffen aus einer Suspension oder Suspensionen mit mindestens einem durchströmten Filtermittel, wobei auf der Retentatseite des Filtermittels ein Mittel zur Erzeugung eines Scherfeldes angeordnet ist, das ein annähernd homogenes Scherfeld und Druckgefälle über den Einströmquerschnitt des Filtermittels erzeugt. Das Mittel zur Erzeugung des Scherfeldes kann durch einen rotationssymmetrischen Rotor gebildet werden. Als Filtermittel können Matrizen verwendet werden, die zur Adsorption der abzutrennenden Gelöststoffe geeignet sind. Dabei kommen oberflächenmodifizierte Membranen, beispielsweise Affinitätsmembranen, in Betracht. Die Filtervorrichtung kann einstufig, zweistufig oder mehrstufig sein.

FIG. 1

a)

**Beschreibung**

Die Erfindung betrifft eine Vorrichtung zur schonenden kontinuierlichen Abtrennung und Aufreinigung von Gelöststoffen aus Suspensionen, insbesondere biologisch aktiven Proteinen aus Zellsuspensionen oder Kulturen, unter Verwendung des Prinzips der Affinitätsfiltration mit oberflächenmodifizierten Membranen.

Bekannte Verfahren zur Isolierung und Reinigung von Proteinen aus Zellsuspensionen beinhalten häufig eine Vielzahl von Schritten, die sich prinzipiell in vier Untergruppen einteilen lassen: 1. Zellabtrennung und Konzentrierung, 2. Voranreicherung, 3. Feinreinigung und 4. Polishing. Hinsichtlich Produktausbeute und -stabilität sind insbesondere die Stufen 1 und 2 kritisch zu bewerten. Typische Verfahren in diesen Stufen sind Zentrifugation, Mikrofiltration, Ultrafiltration, Fällung und Extraktion. Insbesondere bei der Herstellung von biologisch aktiven Proteinen mit Hilfe von tierischen Zellkulturen stellt sich die Forderung nach einer schonenden kontinuierlichen Zellabtrennung und Anreicherung der Zielprodukte. Die hier häufig eingesetzten herkömmlichen Tangentialflußverfahren, wie beispielsweise Mikrofiltration und Ultrafiltration, liefern zwar ein partikelfreies Filtrat, sind aber in ihrer Leistung hinsichtlich Fluß, Standzeit und Produktausbeute wegen der bekannten Probleme des Membranfouling limitiert und erfordern eine weitere Produktbehandlung zur Anreicherung, wie z.B. Chromatografie. Andere Verfahren zur Zellabtrennung, wie die Zentrifugation, liefern in der Regel keinen partikelfreien Überstand, was die folgenden Chromatografieschritte wiederum limitiert.

Aus ökonomischer und verfahrenstechnischer Sicht ist die Reduzierung der Anzahl der Aufarbeitungsschritte wünschenswert. Ein Ansatz dazu sind Verfahren, die die Stufen Zellabtrennung, Voranreicherung und Konzentrierung zusammenfassen. Derzeit sind drei Varianten beschrieben, dies sind a) der Einsatz der Membran-Affinitätsfiltration, b) der Einsatz von Fluidized/Expanded Verfahren und c) der Einsatz von Big-Beads in konventionellen säulenchromatografischen Verfahren. Prinzipiell sind diese Verfahren in der Lage feststoffbelastete oder zellhaltige Medien zu verarbeiten, aber nur das Membranfiltrationsverfahren liefert ein partikelfreies Filtrat und ist als einziges zur kontinuierlichen Zellrückführung geeignet.

Die Membranaffinitätsfiltration wurde bisher überwiegend zur Behandlung partikelfreier Lösungen eingesetzt, wie z.B. zur Anreicherung von pharmazeutischen Proteinen aus Kulturlösungen (Brandt, S., Goffe, R.A., Kessler, S.B., O'Connor, J.L., Zale, S.E.: "Membrane-Based Affinity Technology for Commercial Scale Purifications", Bio/Technology 6, 779-782 (1988)). Die verwendeten Membranmodule sind typischerweise konventionelle Tangentialflußsysteme, wie z.B. Hohlfasern, oder Filterstapel, die zur Direktfiltration oder Membranchromatografie (Langlotz, P., Krause, S., Kroner, K.H.: "Affinitätsmembranen für die Bioproduktaufarbeitung", F&S Filtrieren u. Separieren, 5 (2) 62-70 (1991) und Thömmes, J., Kula, M.R.: "Membrane Chromatography - An Integrated Concept in the Downstream Processing of Proteins", Biotechnol.Progress, 11, 357-367 (1995)) verwendet werden. Die Behandlung partikelhaltiger Suspensionen ist bisher nur ansatzweise beschrieben (Kroner, K.H.: "Cross-Flow Application of Affinity Membranes", Membrane Processes in Separation and Purification, NATO ASI Series E, Applied Sciences, Vol.272, Kluwer Academic Publishers, Dordrecht (1994)).

Ein besonderes Problem des Membranverfahrens ist das Membran-Fouling, d.h. die Verblockung oder Belagsbildung an der Membran, wodurch die Leistung und insbesondere auch die trennspezifischen Eigenschaften, wie die Trenngrenze - im Falle von Affinitätsmembranen auch die Adsorptionscharakteristik - negativ beeinflußt werden (Langlotz, P., Krause, S., Kroner, K.H.: "Affinitätsmembranen für die Bioproduktaufarbeitung", F&S Filtrieren u. Separieren, 5 (2) 62-70 (1991) und Kroner, K.H.: "Cross-Flow Application of Affinity Membranes", Membrane Processes in Separation and Purification, NATO ASI Series E, Applied Sciences, Vol.272, Kluwer Academic Publishers, Dordrecht (1994)). Zur Reduzierung dieser Probleme ist es üblich die Membranmodule mit sehr hohen tangentialen Überströmungsgeschwindigkeiten zu betreiben, bevorzugt im turbulenten Strömungsbereich. Dies führt aber zu einem hohen longitudalen Druckverlust, der wiederum zu einer ungleichmäßigen Verteilung des Flußes über der Membran führt. Dadurch bedingt kommt es zu einer Verringerung der Membrankapazität, auch bekannt unter dem Begriff "verfrühter Durchbruch" und zu einer Peak-Verbreiterung (Kroner, K.H.: "Cross-Flow Application of Affinity Membranes", Membrane Processes in Separation and Purification, NATO ASI Series E, Applied Sciences, Vol.272, Kluwer Academic Publishers, Dordrecht (1994)). Die Nutzung herkömmlicher Tangentialflußsysteme zur Membranaffinitätsfiltration im chromatografieanalogen Betrieb ist daher nur eingeschränkt möglich. Ein weiteres Problem konventioneller Tangentialflußssysteme ist das Auftreten hoher Scherkräfte, die insbesondere beim Einsatz solcher Module bei der Filtration von tierischen Zellkulturen zu einer Zellzerstörung führen können. Dies führt zur Freisetzung von Zellinhaltsstoffen und Zellbruchstücken, die die Leistung der Membranfiltration weiter limitieren und den Einsatz bei kontinuierlicher Zellrückführung stark einschränken.

Bekannt sind auch Rotationsmodule, wie z.B. rotierende Scheibenfilter, bei denen die Scherung an der Membran mechanisch erzeugt wird. Dies ist beispielsweise beschrieben in Murkes, J. et al: "Crossflow Filtration", Wiley, New York (1988). Diese Module zeigen zwar höhere Filtrationsleistungen als Tangentialflußsysteme; die wirksamen Scherkräfte sind aber ebenfalls höher und radienabhängig ungleichmäßig über dem Filtermittel verteilt: ein Einsatz mit empfindlichen Zellen ist deshalb stark eingeschränkt.

Der Erfindung liegt daher die Aufgabe zugrunde, eine Filtrationsvorrichtung zur integrierten Abtrennung und Anreicherung von Gelöststoffen aus partikelhaltigen Suspensionen zu schaffen, die eine annähernd optimale Ausnutzung der Filterfläche bei gleichzeitiger Schonung des Zellmaterials erreicht und die in einfacher Weise scale-up fähig ist.

Die Aufgabe wird durch die Merkmale des Anspruchs 1 gelöst. Vorteilhafte Ausgestaltungen der Erfindung sind Gegenstand der Unteransprüche.

Die vorliegende Erfindung betrifft eine Vorrichtung zur Abtrennung und/oder Anreicherung von Gelöststoffen aus einer Suspension mit mindestens einem durchströmten Filtermittel, wobei auf der Retentatseite des Filtermittels ein Mittel zur Erzeugung eines Scherfeldes angeordnet ist, das ein annähernd homogenes Scherfeld über den gesamten Einströmquerschnitt des Filtermittels erzeugt.

Vorzugsweise wird das Mittel zur Erzeugung eines Scherfeldes durch einen rotationssymmetrischen Rotor gebildet, wobei der Abstand zwischen Rotor und Filtermittel vorzugsweise radial nach außen zur Kompensierung der erhöhten Umfangsgeschwindigkeit zunimmt und somit das homogene Scherfeldes erzeugt wird. Einen bevorzugte Ausführungsform des rotationssymmetrichen Rotors ist der einfache konische Rotor, bei dem der Abstand (Spaltweite) s eine lineare Funktion des Radius r bildet. Es ist allerdings ausreichend, daß der Rotor nur im Bereich der Filtermembran diese lineare Abhängigkeit zeigt, d.h. der Konus kann auch ein Kegelstumpf sein. Die Filtermembran ist im allgemeinen planar und der Filter kann je nach Einsatz als Querstromfilter betrieben werden.

Vorzugsweise ist das Verhältnis der Spaltweite s zwischen Filtermembran und Rotor und dem Rotorradius r kleiner als 0,2, d.h. $s/r < 0,2$; vorzugsweise $s/r < 0,1$ sowie $> 0,05$. Der Konuswinkel $\phi$ liegt im Bereich $< 16°$, vorzugsweise zwischen 3 und 6°. Vorzugsweise beträgt der Konuswinkel $\phi$ des Rotorkonus ca. 4°.

Möglich ist auch die Abführung des Retentats bzw. Konzentrats durch eine in der Nähe der Achse angeordnete Auslaßöffnung oder durch die Achse selbst, falls die Achse als Hohlwelle ausgebildet ist.

Um die Gesamtfilterfläche und damit die Filtrierleistung der Filtriervorrichtung zu erhöhen, ist es weiterhin möglich auch die Oberfläche des Rotorkonus oder - kegelstumpfs als Filter auszulegen, beispielsweise indem die Oberfläche mit einer entsprechenden Porosität versehen wird. Das erzeugte Scherfeld wirkt in diesem Fall auf beide Oberflächen, nämlich der planaren Oberfläche des eigentlichen Filters und der als Filter gestalteten Oberfläche des Konus. Das in dem in diesem Fall hohlen Rotor anfallende Filtrat kann kann beispielsweise über die oben genannte Hohlwelle abgeleitet werden. Vorteilhafterweise kann so die aktive, zur Verfügung stehende Filterfläche vergrößert werden, ohne daß die Vorrichtung selbst vergrößert werden muß, wobei die Vorteile des Scherfeldes erhalten bleiben.

Aus den erfindungsgemäßen Filtrationsvorrichtungen können durch Hintereinanderschalten, mit anderen Worten in einer Stack-Anordnung, oder Parallelschalten Filtrationssysteme gebildet werden. Mit anderen Worten, die Leistung des Filtrationssystems kann dem Bedarf angepaßt werden.

Vorzugsweise werden als Filtermittel oberflächenmodifizierte Membranen, insbesondere Affinitätsmembranen auf Basis von Mikrofiltrationsmembranen, eingesetzt. Diese Membranen können Einzelschichten oder Stapel von Mehrfachschichten zur Erhöhung der Gesamtkapazität oder Kombinationen (Sandwiches) aus oberflächlichen Trennmembranen und aktivem Packungsmaterial (Gele, Fasern, Papiere o.ä.) sein. Mit dieser Vorrichtung wird es möglich, einen chromatografieanalogen Betrieb mit partikelhaltigen Suspensionen zu realisieren.
Die besonderen Vorteile der erfindungsgemäßen Vorrichtung sind nachfolgend aufgeführt:

Das beschriebene Filtrationssystem erlaubt einen hohen Filtratfluß bei gleichzeitiger Schonung des Zellmaterials, bedingt durch die besondere Hydrodynamik. Bei Verwendung eines konischen Rotors ergibt sich im Gegensatz zu bekannten Rotationsscheibenfiltern ein radienunabhängiges, annähernd gleich-mäßiges Schergefälle über den gesamtem Membranquerschnitt. Dabei kann angenommen werden, daß die Zellen aufgrund des sog. "hydrodynamic lift effects"' (Vasseur, P., Cox, R.G.: "The Lateral Migration of a Sperical Particle in Two-Dimensional Shear Flow", J.Fluid Mech. 78, 385-401 (1976)) von der Membranwand (dem Ort höchster Wandschubspannung) ferngehalten werden und sich überwiegend im Bereich der Kernströmung befinden, wo aufgrund des parabolischen Profils des Geschwindigkeitsgefälles niedrige Scherraten vorliegen. Dieses Verhalten kann in besonderer Weise vorteilhaft bei der Filtration tierischer Zellen ($r_p > 3\mu m$) genutzt werden. Berechnungen zeigen, daß Filtratflüsse von $> 100$ L/hm$^2$ erzielbar sind, u.z. für mehr als 90% der Membranfläche (mit $A=f[r^2]$). Herkömmliche Filtrationssysteme zeigen unter gleichen Bedingungen Filtrationsleistungen typischerweise $<< 100$ L/hm$^2$.

Im Gegensatz zu herkömmlichen Rotationsscheibenfiltern (High-Shear Filtern), die generell im turbulenten Strömungsbereich IV (Murkes, J., Carlsson, C.G.: "Crossflow Filtration", Wiley, New York (1988)) betrieben werden, kann die vorliegende Filtrationsvorrichtung im laminaren Strömungsbereich II (Murkes, J., Carlsson, C.G.: "Crossflow Filtration", Wiley, New York (1988)) betrieben werden, was den unter Punkt 1 ausgeführten "hydrodynamic lift effect" positiv unterstützt.

Im Gegensatz zu herkömmlichen Tangentialflußfiltern, die einen großen strömungsbedingten Druckabfall über der Länge des Membrankanals ( $P = f[w,L,Re]$ ) zeigen, ist der radienabhängige zentrifugale Druckgradient ( $P = [w^2]$ ; $w = \omega * r$ ) im hier vorliegenden System klein und nicht von der Viskosität des Mediums abhängig. Ein Vergleich des längen- oder radienabhängigen Druck- und Flußprofils für ein konventionelles Tangentialflußmodul (Hohlfasermodul) und des erfindungsgemäßen Filtrationsmoduls mit radialer Scherung zeigt, daß sich für das Hohlfasermodul unter den zugrunde gelegten typischen Betriebsbedingungen (w = 2m/s, TMP = 0,1 bar) ein Druckverlust von 0,2 bar unter laminaren Bedingungen und von 0,96 bar unter turbulenten Bedingungen ergibt und für das erfindungsgemäße Modul sich ein nominaler zentrifugaler Druckverlust von nur 40 mbar ergibt. Überraschenderweise zeigten Messungen, daß an der Membran aber nur ca. 1/10 dieser Druckdifferenz wirksam wird, was wahrscheinlich durch eine interne Rückströmung zwischen Rotor und Stator bedingt ist. Somit ergibt sich für das erfindungsgemäße Rotationsmodul ein nahezu konstantes Druckgefälle über der gesamten Membran und damit ein gleichmäßiger Fluß über dem gesamten Einströmquerschnitt des Filtermittels. Entsprechend ergibt sich ein besseres Verweilzeitverhalten für die Durchströmung des Filtermittels und - daraus abzuleiten - eine wesentliche Erhöhung der Ausnutzung der Membrankapazität (>90%) im chromatografieanalogen Betrieb. Im Gegensatz zu Tangentialflußmodulen erlaubt die erfindungsgemäße Vorrichtung deshalb einen annähernd optimalen chromatografieanalogen Betrieb.

Im Gegensatz zu anderen Trennsystemen ergibt die Verwendung der erfindungsgemäßen Vorrichtung ein völlig partikelfreies Filtrat, das problemlos und ohne weitere Behandlung weiteren Reinigungsschritten zugeführt werden kann. Im Gegensatz zu den "Fluidized Bed" - bzw. "Big-Beads" Verfahren erlaubt die erfindungsgemäße Vorrichtung den Betrieb mit kontinuierlicher Zellrückführung, wie z.B. Perfusionsverfahren mit tierischen Zellkulturen. Im Gegensatz zu Tangentialflußmodulen und anderen bekannten Rotationsscheibenfiltern erlaubt die erfindungsgemäße Vorrichtung eine effiziente und schonende Zellabtrennung unter Erhalt der Zellvitalität, insbesondere auch von empfindlichen Zellkulturen.

Das scale-up der erfindungsgemäßen Vorrichtung kann in einfacher Weise erfolgen durch:

a) Die Verwendung von mehr als einer Filterschicht zur Erhöhung der Kapazität, dazu können Membran- oder Filtermittelstapel eingesetzt werden (analog zu einer Säulenpackung in der Chromatografie).

b) Die Verwendung von mehr als einer Filterschicht durch mehrstufige Anordnung nach dem Rotor-Stator Prinzip, dabei wird zum Beispiel ein Rotationselement von zwei Filtermitteln begrenzt.

c) Vergrößerung des Radius der Filterzelle, dabei ist bei konstanter Umfangs-geschwindigkeit ( $w$ = const. = $\omega^* r$ ) der radiale Druckgradient längenunabhängig, d.h. für Filter mit unterschiedlichem Durchmesser gleich, im Gegensatz zu Tangentialflußmodulen, bei denen bei konstanter Überströmungsgeschwindigkeit der Druckverlust mit der Länge zunimmt.

Bevorzugte Ausführungsformen der Erfindung werden anhand der beigefügten Zeichnungen beschrieben, in denen:

Fig. 1 den allgemeinen Aufbau und das Arbeitsprinzip einer einstufigen (a), zweistufigen (b) und mehrstufigen (c) erfindungsgemäßen Vorrichtung zeigt,

Fig. 2 die erfindungsgemäße Vorrichtung in Verbindung mit verschiedenen Filtermittelalternativen zeigt, nämlich Mehrschichtaufbau Stapel (a), Mehrschichtaufbau Sandwich (b) und Filtersäule (c),

Fig. 3 den prinzipiellen Betrieb der Vorrichtung zur Abtrennung und Anreicherung von Gelöststoffen aus partikelhaltigen Suspensionen im chromatografieanalogen Betrieb mit den Schritten a) Beladen, b) Waschen und c) Eluieren zeigt.

Fig. 4 eine schematische perspektivische Darstellung einer einstufigen Filtrationsvorrichtung zeigt,

Figuren 5A und 5B eine schematische Querschnittsdarstellung und eine Draufsicht einer zweistufigen Querstromfiltrationsvorrichtung zeigt,

Fig. 6 eine schematische Darstellung des Geschwindigkeitsgefälles im Spalt zwischen Rotor und Filtermittel zeigt,

Fig. 7 eine schematische Darstellung des Lifteffekts zeigt,

Fig. 8 eine Darstellung des Grenzflußes nach der Theorie des "Hydrodynamic lift effects" in einer bevorzugten Ausführung der Filtrationsvorrichtung zeigt,

Fig. 9 einen Leistungsvergleich der erfindungsgemäßen Vorrichtung mit konventionellen Systemen zeigt,

Fig. 10 einen Vergleich der Zellvitalität der erfindungsgemäßen Vorrichtung mit der Vitalität konventioneller Systeme bei gleicher Wandschubspannung zeigt,

Fig. 11A und 11B eine Vergleich des Druck- und Flußverlaufs über der Filterfläche für ein konventionelles Tangentialflußmodul (Hohlfaser) und der erfindungs-gemäßen Filtrationsvorrichtung zeigen,

Fig. 12 einen Vergleich der Verweilzeitverteilung für die Durchströmung des Filtermittels für ein Hohlfasermodul und die erfindungsgemäße Vorrichtung zeigt,

Fig. 13 eine Rührzelle mit konischem Rotor zur absatzweisen Filtration zeigt,

Fig. 14 eine Parallelanordnung mehrerer erfindungsgemäßer Vorrichtungen zeigt, und

Fig. 15 eine Seriellanordnung mehrerer erfindungsgemäßer Vorrichtungen zeigt.

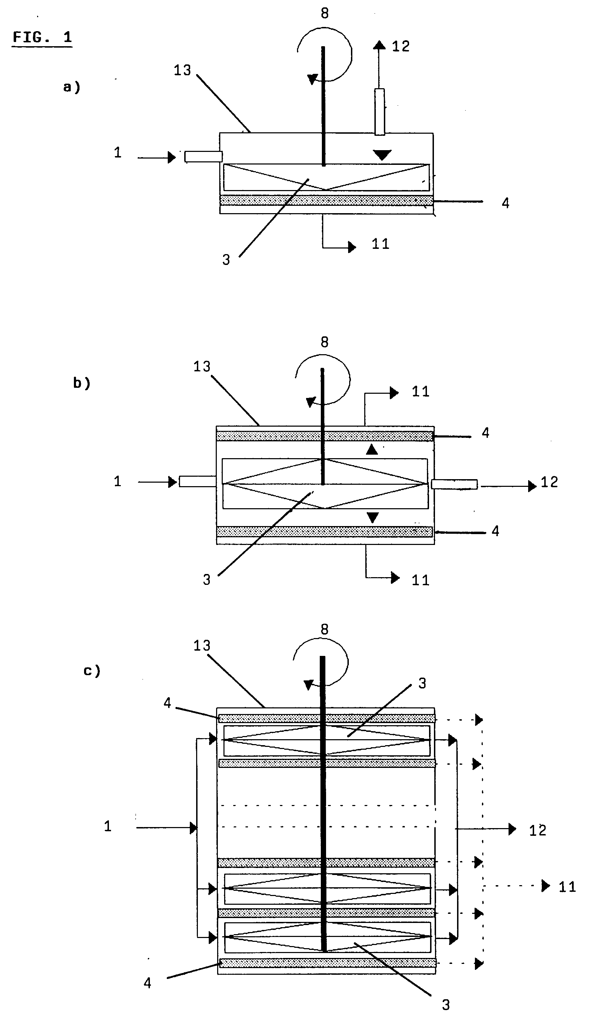

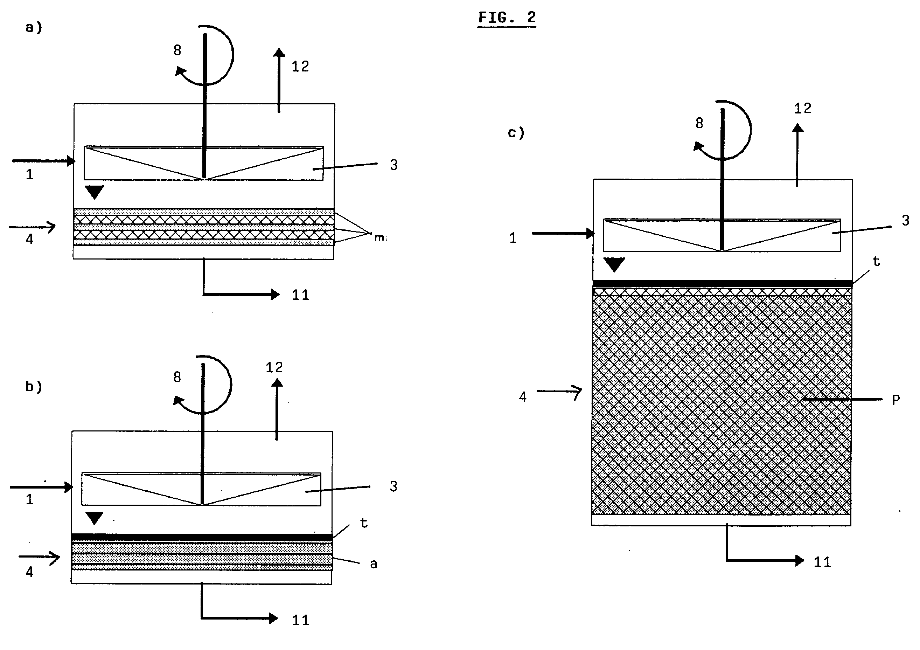

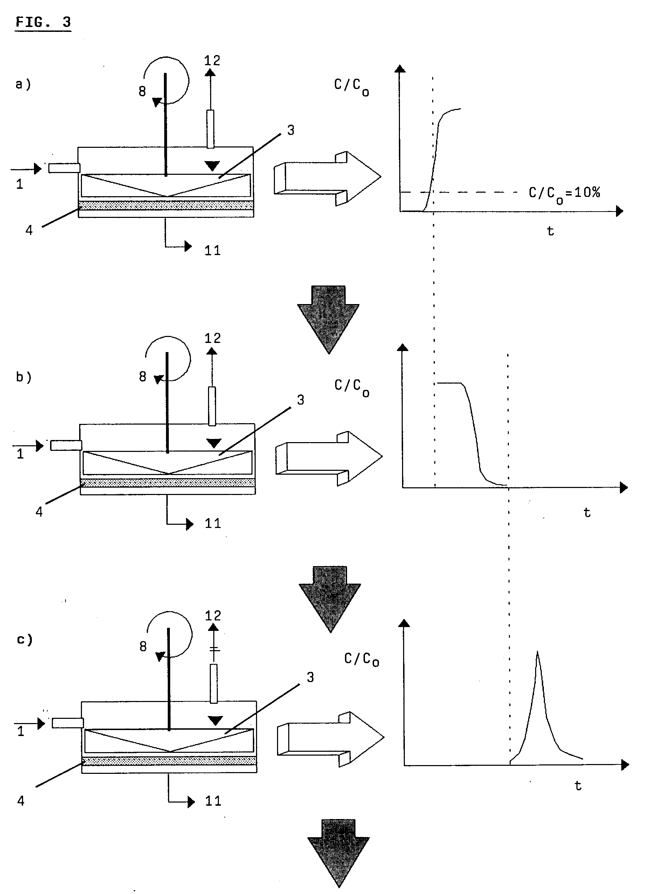

Fig. 1 Zeigt ein in einem vorzugsweise zylindrischen Gehäuse 13 angeordnetes Mittel 3 zur Erzeugung eines homogenen Scherfeldes, wobei vorzugsweise ein rotationssymmetrischer Rotor 3, insbesondere ein kegelförmiger Rotor 3, gegenüber einem Filtermittel 4 angeordnet ist, der für eine gleichmäßige Überströmung des Filtermittels 4 zur Verhinderung einer Belagsbildung an dem Filtermittel 4 sorgt. Der Rotor 3 wird dabei um seine Symmetrieachse 8 gedreht. Die zu filtrierende Suspension wird mit einer geeigneten externen Einrichtung, beispielsweise einer Pumpe, oberhalb des Filtermittels 4 in das Modul eingespeist am Punkt 1 und verläßt das Modul über den Retentatauslaß 12. Durch Anlegen eines äußeren Drucks wird über das Filtermittel ein gleichmäßiger Filtratstrom erzeugt, der über den Filtratauslaß 11 abgeführt wird. Die Betriebparameter Druck, Scherung (Drehzahl) und Suspensionszufuhr sind frei wählbar. Das Filtermittel 4 kann eine beliebige Filterschicht sein, vorzugsweise eine Membran, die eine vollständige Partikelrückhaltung gewährleistet oder eine oberflächenmodifizierte Membran, die gleichzeitig zur Adsorption des Zielprodukts eingesetzt werden kann. Die einfachste Ausführung ist eine einstufige Anordnung mit einem Rotor 3 und einem Filtermittel 4(a). Zur Vergrößerung der Filterfläche und unter Ausnutzung der gesamtem Rotorkammer können sowohl ober- als auch unterhalb des Rotors 3 Filtermittel 4 eingesetzt werden, der Rotor 3 hat auf beiden Seiten die gleiche Form - zweistufige Ausführung (b). Zur weiteren Vergrößerung der Filterfläche kann auf Basis der zweistufigen Anordnung auch eine mehrstufige Anordnung nach dem Rotor-Stator Prinzip aufgebaut werden (c). Hier wird jedes Rotorkammerelement durch zwei Filtermittel 4 begrenzt. Die Dreiecke in der Fig. 1 zeigen die Richtung des Flusses an. Dasselbe gilt für die Figuren 2 und 3.

Fig. 2 zeigt die Verwendung alternativer Filtermittel 4 in der erfindungsgemäßen Vorrichtung. Neben der einlagigen Konfiguration, z.B. Filtermembran, kann zur Kapazitätssteigerung ein mehrlagiger Aufbau gewählt werden, z.B. ein Stapel oder Paket mehrerer Membranen, deren Anzahl n = i beträgt, insbesondere bifunktionelle Membranen wie Affinitätsmembranen (a). Daneben kann eine Konfiguration mit Kombination einer Trennschicht oder -Membran t (z.B. Mikrofiltermembran) und einer oder mehereren nachgeschalteten aktiven Schichten a gewählt werden (b). In einer besonderen Ausführungsforn dieser Variante kann die aktive Schicht auch eine Packung oder ein Bett P, bestehend aus einem Chromatografiematerial, sein. Dabei wird die Packung P von einer Trennschicht/-Membran t abgedeckt. Diese Form stellt im Prinzip eine Chromatografiesäule mit integriertem Filtersystem dar (c).

Fig. 3 zeigt die prinzipielle Arbeitsweise der Filtervorrichtung, bestehend aus einem Einlaß 1, einem um eine Achse 8 drehbaren Rotor 3, einem Filtermittel 4 und Filtratauslaß 11 sowie Retentatauslaß 12, im chromatografieanalogen Betrieb. Ferner ist der zeitliche Verlauf der Filtratkonzentration normiert auf die Ausgangskonzentration dargestellt. Der Ablauf ist wie folgt: a) Beladung: Die partikelhaltige Suspension wird mittels einer externen Pumpe durch das Modul geleitet und bei Bedarf in die Suspensionsvorlage (z.B. Fermenter) zurückgeleitet. Unter Anlegen eines spezifischen Filtrationsdrucks wird ein partikelfreier Filtratstrom über die aktive Filterschicht erzeugt. Dieser Filtratstrom wird solange aufrecht erhalten, bis ein Produktdurchbruch im Filtrat sichtbar wird. Ein typischer Richtwert beträgt max. ca. 10% der Ausgangskonzentration, d.h. $C/C_o$ = 10 %. Das Monitoring kann mittels einer geeigneten analytischen Methode erfolgen, z.B. photometrisch. b) Waschen: Die Suspensionszufuhr wird abgestellt und eine geeignete Spüllösung, z.B. ein Puffer, unter laufender Filtration durch das Modul geleitet, bis die Signalhöhe im Filtrat gegen Null geht. c) Elution: Zur Elution der an der aktiven Schicht gebundenen Stoffe wird ein geeigneter Eluent, beispielsweise ein Puffer, in das Modul geleitet u.z. in der Weise, daß zunächst die Spüllösung über den Retentatausgang verdrängt wird und dann, nach schließen dieses Ausgangs, der Eluent direkt durch das Filtermittel geleitet wird, dabei kann eine Stufen- oder Gradientenelution erfolgen, in ähnlicher Weise wie bei der Chromatografie. Das eluierte Produkt kann in bekannter Weise aufgefangen oder fraktioniert werden. Nach Abschluß des gesamten Zyklusses kann, ggfs. nach einer weiteren Spülung mit Äquilibrierpuffer, das Modul für den nächsten Zyklus wieder auf Suspensionszufuhr geschaltet werden.

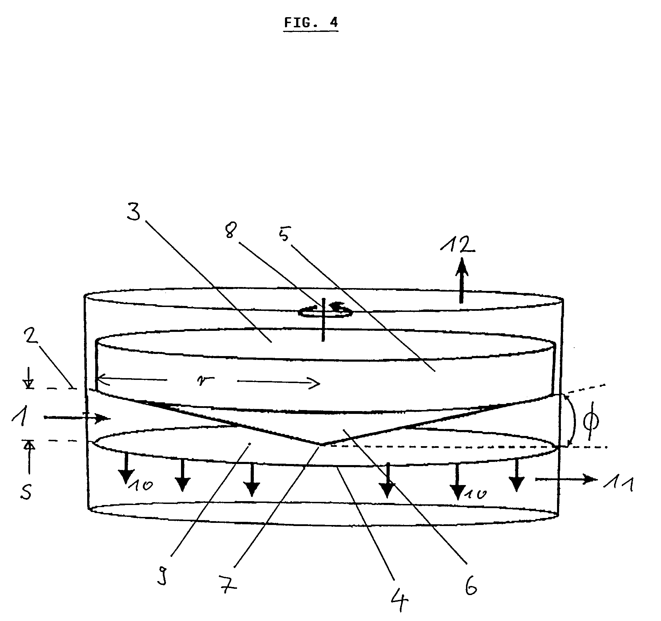

Fig. 4 zeigt eine perspektivische, schematische Darstellung einer bevorzugten Ausführungsform der erfindungsgemäßen Filtrationsvorrichtung. Ein Zuführfluß 1 der zu filtrierenden Flüssigkeit oder Suspension tritt in den Spalt 2 mit der Spaltbreite s ein, wobei der Spalt 2 durch einen Rotor 3 und ein Filtermittel 4 definiert ist. Das Filtermittel 4 wird durch eine planare Filtermembran gebildet. Der Rotor 3 besteht aus einem zylinderförmigen Körper 5 und einem Konusabschnitt 6, dessen Spitze 7 zur Filtermembran 4 hin zeigt. Der Rotor 3 rotiert um seine Symmetrieachse 8.

Infolge dieser Rotation entsteht aufgrund des Konusabschnitts 6 des Rotors 3 ein konstantes Schergefälle über der gesamten Filterfläche 9 des Filtermittels 4. Das erzeugte homogene Scherfeld über der Filterfläche 9 ist eine Funktion des Öffnungswinkels _ des Konus 6 in bezug auf die Filterfläche 9, des Abstandes $s_0$ der Konusspitze gegenüber der Filterfläche 9, der Spaltbreite s, die durch den Abstand zwischen Filterfläche 9 und Zylinderkörper 5 gebildet wird, sowie des Verhältnisses Spaltbreite zu Konusradius s/r. In einer bevorzugten Ausführungsforn beträgt der Rotorradius 35 - 150 mm, der Abstand Kegelspitze Konus zu Membran $s_0$ ist variabel und liegt zwischen 0 und 5 mm, vorzugsweise 1 mm der Kegelwinkel $\phi$ des Rotors liegt zwischen 3° und 6°, vorzugsweise 4°. Ferner ist das Verhältnis Spaltweite s zu Rotorradius r (s/r) kleiner als 0,1 und größer als 0,05. Die Flüssigkeit tritt in der bevorzugten Ausführungsform in axialer Durchtrittsrichtung 10 durch das Filtermittel 4 durch und wird als Filtrat 11 quer zur Durchtrittsrichtung 10 entnommen. Das zurückgehaltene Retentat 12 fließt in senkrechter Richtung von der Filteroberfläche 9 weg ab. Als Membranen für das Filtermittel 4 kommen handelsübliche Filtermembranen sowie funktionelle Membranen wie beispielsweise Affinitätsmembranen, in Betracht. Die Größe des Rotors 3 und der Filterfläche 9 hängen von dem beabsichtigten Verwendungszweck sowie dem zu erzielenden Durchfluß, d.h. der zu erzielenden Filterleistung, ab. Derartige Filter können beispielsweise zur Erhöhung der Filterleistung in einem System parallel angeordnet oder seriell als Stack angeordnet werden. Ferner muß die erfindungsgemäße Filtrationsvorrichtung nicht als Querstromfilter ausgelegt sein, es ist auch möglich, die zu filtrierende Lösung in axialer Richtung 10 der Symmetrieachse 8 dem Filtrationsmittel 4 zuzuführen. Dabei würde dann das Retentat 12 quer zur Zuführrichtung entnommen werden. Der Antrieb des Rotors 3 wird entsprechend dem Verwendungszweck gewählt. So kann der Rotor beispielsweise direkt über einen Elektromotor angetrieben werden oder als magnetodynamischer Rührer ausgelegt sein.

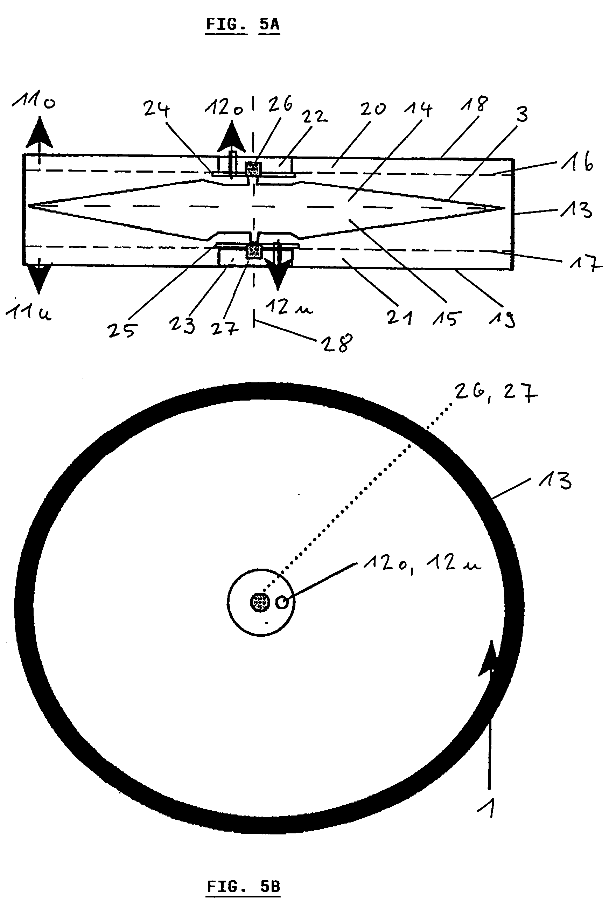

Die Fig. 5A zeigt eine Doppelmembranrührzelle mit einem Gehäuse 13, in dem ein Rotor 3 angeordnet ist. Der Rotor 3 besteht aus einem oberen und einem unteren Konusabschnitt 14, 15 derart, daß ein beidseitig nach außen gerichteter Doppelkonus entsteht. Beidseitig des als Doppelkonus ausgelegten Rotors 3 sind Filtermittel 16, 17 in dem gehäuse 13 angeordnet, so daß zwischen der entsprechenden unteren und oberen Deckwand 18, 19 des Gehäuses 13 ein jeweils Raum 20, 21 zur Aufnahme des jeweiligen Filtrats $11_o$ und $11_u$ verbleibt. Ein oberer und unterer Stutzen 22 bzw. 23 ragt axialsymmetrisch in den durch das Gehäuse 13 gebildeten Raum hinein, wobei jeder Stutzen 22, 23 einen entsprechenden Deckel 24, 25 aufweist. In dem Deckel ist jeweils ein Lager 26 bzw. 27 zur axialen Aufnahme der Achse 28 des Rotors 3 vorgesehen. Im Bereich der Lagerstutzen 22, 23 weist der Rotor 3 keine Konusform mehr auf, sondern ist entsprechend abgeflacht, da hier kein Scherfeld auf einer Membran 16, 17 erzeugt werden muß. Mit anderen Worten, der Konus 14, 15 hat im wesentlichen die Form eines Kegelstumpfes, da im Bereich der nach innen ragenden Stutzen 22, 23 kein Scherfeld erzeugt weden muß, da hier keine Filtermembran vorhanden ist. Ferner ist in jedem Deckel 24, 25 des entsprechenden Stutzens 22, 23 jeweils mindestens ein Retentatauslaß $12_o$ und $12_u$ vorgesehen, der seitlich von der Symmetrieachse seitlich versetzt ist.

Fig. 5B zeigt die Doppelmembranrührzelle in Draufsicht. Diese umfaßt die äußere Wand des zylindrischen Gehäuses 13. Axialsymmetrisch dazu ist der Stutzen 22 bzw. 23 angeordnet mit dem axialen Lager 26 bzw. 27 und dem Retentatauslaß $12_o$ bzw. $12_u$` Die Zuführung 1 der zu klärenden Lösung bzw. Suspension erfolgt in dieser Ausführungsform in tangentialer Richtung, beispielsweise durch ein entsprechend ausgebildetes Zulaufrohr (nicht dargestellt).

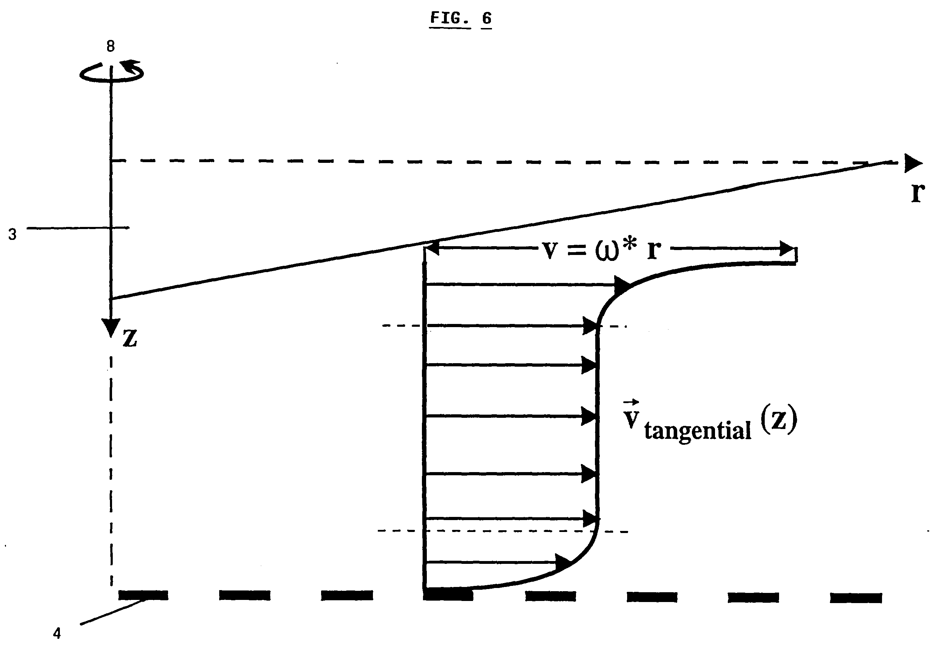

Fig. 6 zeigt eine schematische Darstellung des postulierten tangentialen Geschwindigkeitsgefälles an der Membran im Spalt zwischen Rotor und Stator ($w = \omega^* r$, z = achsiale Richtung) mit hyperbolischen Verlauf: Geschwindigkeitsgradient an der Wand > Geschwindigkeitsgradient im Zentrum.

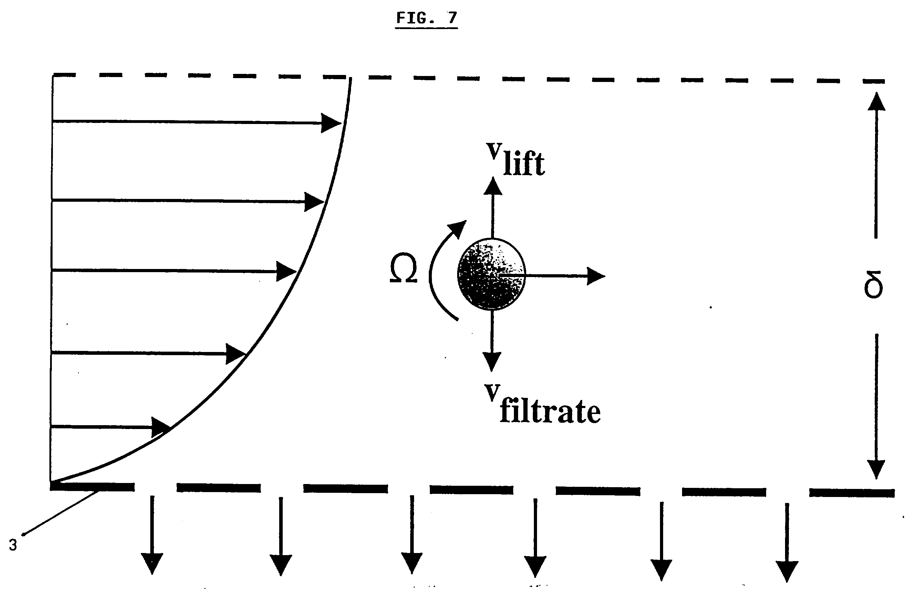

Fig. 7 zeigt eine Darstellung des "Lift-Force" Effekts an einem sphärischen Partikel in einer Querströmung parallel zu einer Wand (Membran); $\Omega$ = Rotation des Partikels, ausgelöst durch die Geschwindigkeitsunterschiede in der Partikelanströmung, $\delta$ = Distanz von der Wand, $v_{lift}$ = äquivalente volumeterische Aufwärtsgeschwingkeit (Lift) des Partikels, $v_{filtrate}$ = äquivalente volumetrische Abwärtsgeschwindigkeit des Partikels. Im Gleichgewichtssustand ist $v_{lift} / v_{filtrate} = 1$, d.h. die Aufwärtsströmung ist gleich der Filtratströmung und das Partikel nimmt eine konstante Lageposition in senkrechter Richtung zur Membran ein.

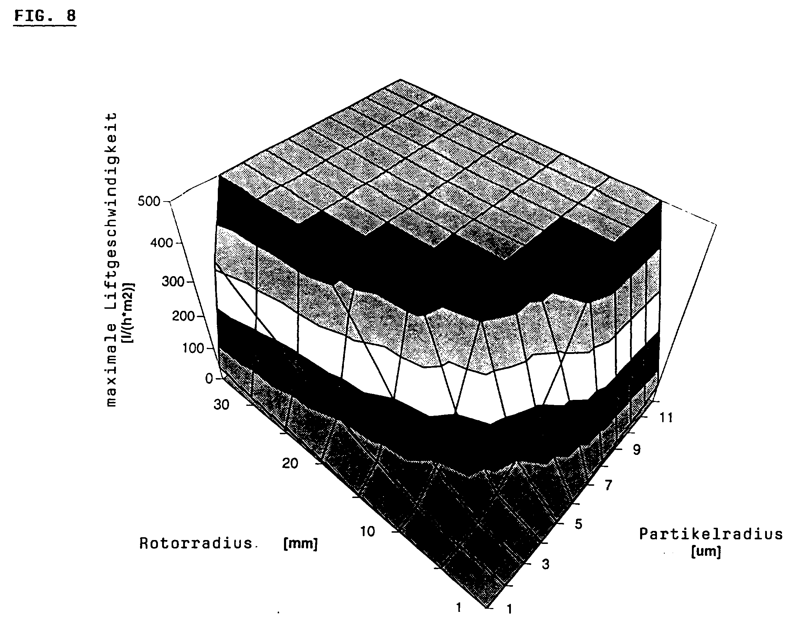

Fig. 8 zeigt berechnete Grenzflüsse für die experimentelle Ausführung der Filtrationsvorrichtung nach der Theorie des 'Lift-Force' Effekts, in Abhängigkeit von Rotorradius und Partikelradius.

Berechnungsansätze:

$$v_{lift} = a^3 * V_w^2 * 0{,}095/v$$

$$Y_w = T_w /\eta ; \text{ mit}$$

$$T_w = 1{,}81^*\rho^*v^{0,5} * (K^*\omega)^{1,5} * r$$

mit a = Partikelradius, $Y_w$ = Wandscherrate, v = kin. Viskosität, $\eta$ = dyn. Viskosität, $\rho$ = Dichte, $\omega$ = Winkelgeschwindigkeit, K = Reibungsbeiwert.

Parameter:

- Drehzahl 900 upm ($\omega$ = 94,25 m/s)
- Dichte 1000kg/m$^3$
- dyn. Viskosität 1,36 mPas
- K = 0,4

Das Berechnungsbeispiel zeigt, daß ab einem Partikelradius > 3 µm, wie er für tierische Zellkulturen typisch ist, ein Grenzfluß von > 100 L/hm$^2$ für den größten Teil der Filterfläche (>90% bei r > 9mm, mit A=f(r$^2$) erzielbar ist.

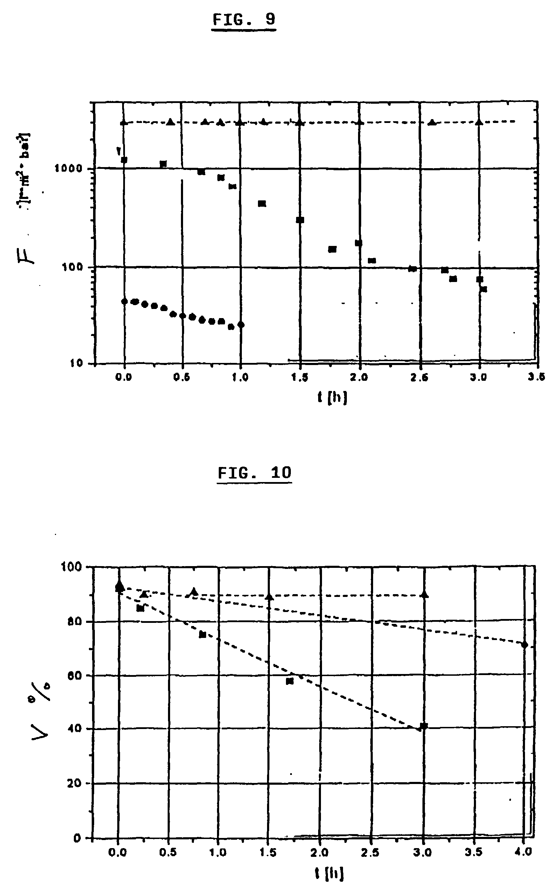

Fig. 9 zeigt einen Leistungsvergleich der erfindungsgemäßen dynamischen Mikrofiltrationsvorrichtung mit konventionellen Systemen. Dabei sind die Messungen der erfindungsgemäßen Vorrichtung mit kleinen Dreiecken, die einer BIOPEM-Einheit mit kleinen Vierecken und die einer konventionellen Querstromeinheit mit schwarzen ausgefüllten Kreisen gekennzeichnet (ebenso bei Fig. 10). Bei allen drei Vorrichtungen wird mit einer Wandschubspannung von 3 N/m$^2$ gearbeitet. Aufgetragen ist der drucknormierte Flux in Einheiten l $\cdot$ h$^{-1}$ $\cdot$ m$^{-2}$ $\cdot$ bar$^{-1}$ gegenüber der Zeit t in Stunden. Dabei ist zu beachten, daß der drucknormierte Flux logarithmisch dargestellt ist. Deutlich zu erkennen ist die Überlegenheit der erfindungsgemäßen Vorrichtung, die ein konstantes Verhalten, d.h. kein Leistungsabfall, über 3,5 Stunden aufzeigt. Mit anderen Worten, der Filtrationsfluß durch den Filter zeigt nach 3,5 Stunden keine Abschwächung. Demgegenüber ist deutlich zu erkennen, daß sowohl die BIOPEM-Filtrationseinheit als auch die bekannte Querstromeinheit einen exponentiellen Abfall zeigen. So weist die BIOPEM-Einheit nach 3 Stunden Standzeit weniger als 10 % der ursprünglichen Leistung auf, während die Querstromeinheit nach bereits einer Stunde nur noch geringen Durchfluß durch den Filter aufgrund von Clogging und Fouling aufweist.

Ein ähnliches Bild ist dem Leistungsvergleich der Fig. 10 zu entnehmen. Dargestellt in der Fig. 10 ist die Vitalität in % gegenüber der Zeit t in Stunden. Auch hier ist zu erkennen, daß die Vitalität bei der erfindungsgemäßen Vorrichtung einen konstanten Verlauf zeigt und nach 3 Stunden Betriebszeit keinen Abfall aufweist. Demgegenüber sinkt die Vitalität bei der Querstromeinheit von 90 auf 70 % in 4 Stunden, während bei der BIOPEM-Filtrationsvorrichtung nach 3 Stunden nur noch eine Vitalität von 40 % der Zellen vorliegt.

Weiterhin müssen zur Erzielung einer stabilen Filtratleistung und damit ausreichender Standzeiten in konventionellen Perfusionssystemen niedrige Flußraten gewählt werden. Dies macht den Einsatz großer Membranflächen notwendig, was ein Hauptkostenfaktor bei klassischen Membranverfahren ist. Tabelle 1 zeigt die erreichbaren stabilen Fluxwerte für bekannte und die erfindungsgemäße Filtrationsvorrichtungen.

Tabelle 1

| | Querstromeinheit (Prostak, Millipore) | Internes Membranperfusionsmodul | Erfindungsgemäß beschriebene Vorrichtung |
|---|---|---|---|
| Flux [1*h$^{-1}$*m$^{-2}$] | 2 - 6 | 2 - 10 | 30 - 50 |

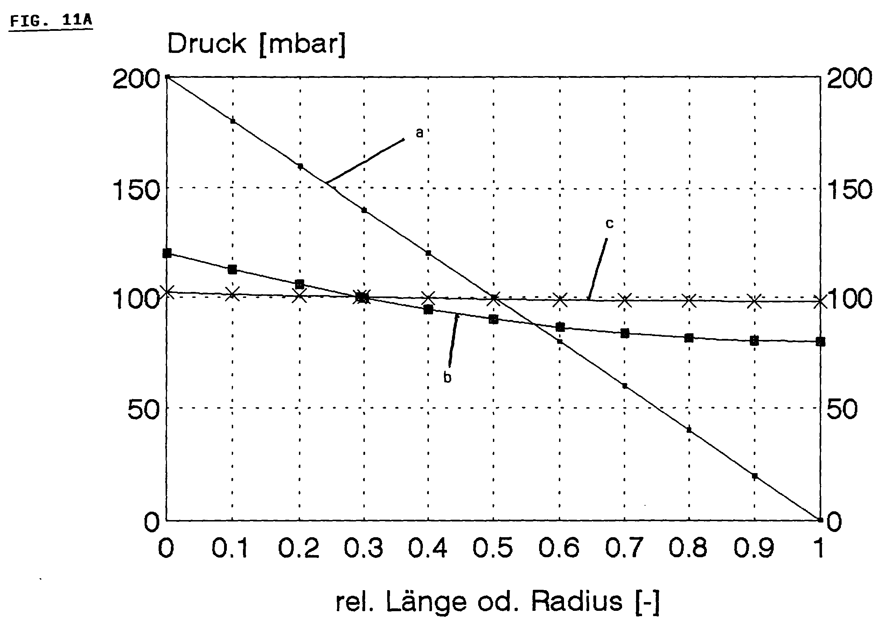

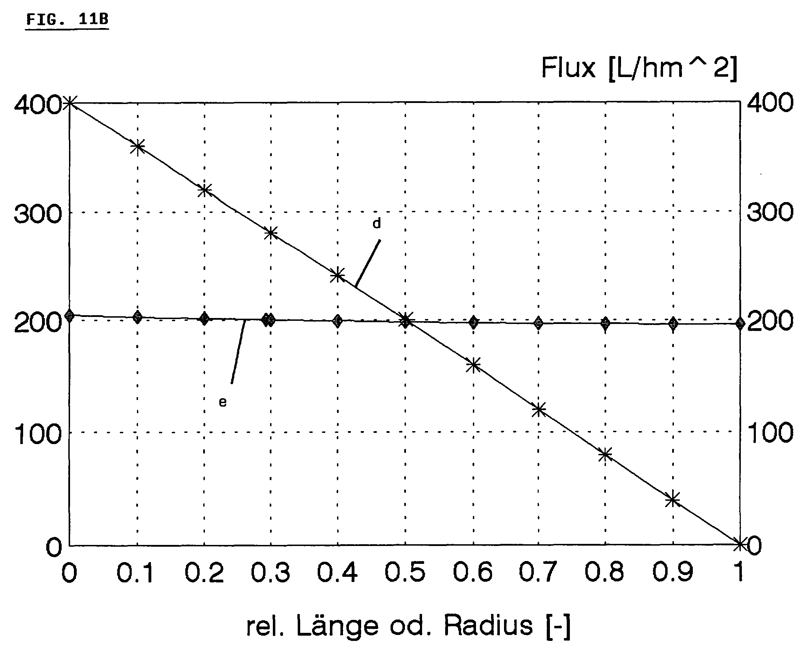

Die Figuren 11A und 11B zeigen den Druck- und Flußverlauf für Wasser über der charakteristischen Länge der Filterfläche im Vergleich zwischen einem konventionellen Tangentialflußmodul (Hohlfaser) und der erfindungsgemäßen Filtrationsvorrichtung. Berechnungsansätze:

$$\Delta P = \lambda \, * \, 0,5 \, * \, \rho \, * \, w^2 \, * \, L \, / \, d_h \text{ (Hohlfaser)} \tag{1}$$

mit $\lambda$ = 64/Re (laminar) und $\lambda$ = 0,3164 * Re$^{-0,25}$ (turbulent)

$$\Delta P = \omega^2 \, * \, r^2 \, * \, \rho = w^2 \, * \, \rho \text{ (Rotationsfilter)} \tag{2}$$

Der Flux für beide Systeme errechnet sich zu Flux = Pm * TMP$_{eff}$, mit Pm = 2000 L/hm$^2$bar und TMP$_{eff}$ = TMP$_{mitt}$ +/- $\Delta P$ (TMP=Transmembrandruck)

Randbedingungen:

Gleiche konst. Strömungs- oder max. Umfangsgeschwindigkeit, TMP$_{mitt}$ = 0,1 bar;

Hohlfaser: L = 30 cm, d$_h$ = 1mm, A = 700 cm$^2$ = 75 Fasern, w = 2m/s , Re = 2000 (lam);
Rotationsmodul: r = 15 cm, A = 700 cm$^2$, w$_{max}$ = 2m/s, $\omega$ = 13,33;

Fig. 11A:

Druckverlauf für (a) Hohlfasermodul über der rel. Länge in mbar. Der mittlere TMP von 0,1 bar wird bei L/2 erreicht (linearer Druckverlustgradient)

Druckverlauf für (b) Rotationsmodul (Zentrifugaldruck nominell) über der rel. Länge (Radius). Der mittlere TMP wird bei L= 0,293 erreicht (1- L/2)

Tatsächlicher Druckverlauf für (c) Rotationsmodul über der rel. Länge.

Das Beispiel zeigt, daß für das Rotationsmodul ein über der Filterfläche annähernd konstanter Wirkdruck herrscht, im Gegensatz zum Hohlfasermodul. Für das scale-up ergibt sich ein weiterer Vorteil daraus, daß bei Annahme von $w = \omega * r = $ konst. , $\Delta P$ konstant bleibt, im Gegensatz zu einem Tangentialflußmodul, wo $\Delta P$ mit der Länge zunimmt.

Fig. 11B:

Flußverlauf für (d) Hohlfasermodul und (e) Rotationsmodul über der rel. Länge in $L/hm^2$. Ausgehend von dem annähernd konstanten Wirkdruck über der Membran ergibt sich für das Rotationsmodul auch ein annähernd konstanter Fluß über dem gesamten Querschnitt der Membran und damit eine gleichmäßige Durchströmung der Filtermatrix.

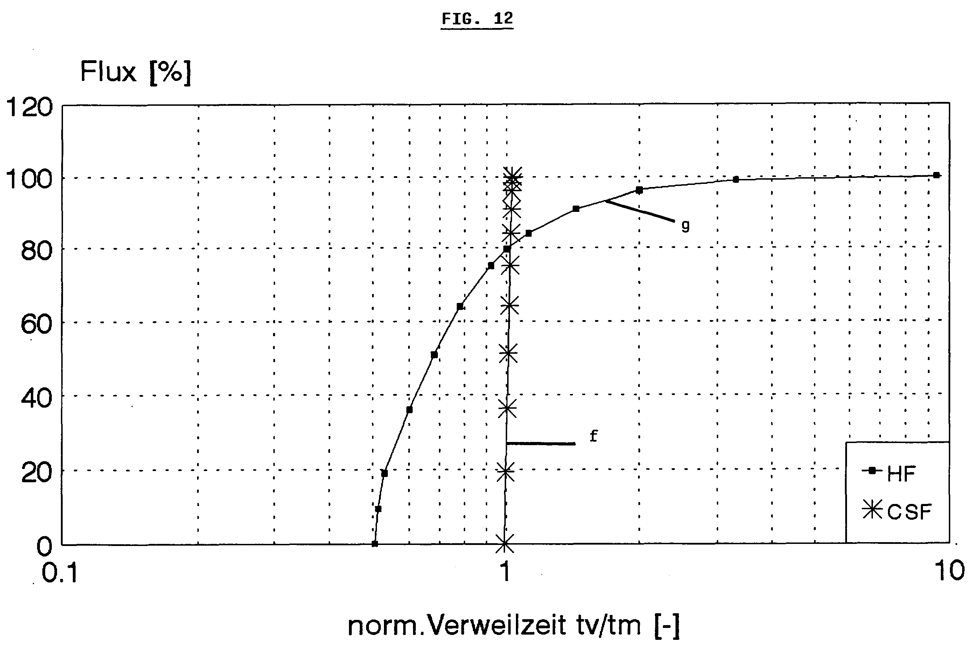

Fig. 12 zeigt einen Vergleich der Verweilzeitverteilung für die Durchströmung des Filtermittels für ein Hohlfasermodul (f) und das erfindungsgemäße Modul (g). Parameter und Bedingungen wie in Abb.10.

Auftragung: Relativer flächenbezogener Flußanteil (%) vs. normierter Verweilzeit. (a) Hohlfasermodul, (b) Rotationsmodul.

Definitionen: Flux (%) = Örtlicher Fluß über der Membranlänge (s.Abb. 9) / mittl. Fluß; normierte Verweilzeit = Flußbezogene Verweilzeit $(t_v)$/ mittlere Verweilzeit $(t_m)$ ; $t_v$=Filtermittel-(Membran) Dicke ($\delta$) / Flux ; $t_m$ = ($\delta$) / mittl. Fluß .

Annahmen: mittl.Fluß = Pm * TMP = 200 l/hm$^2$ = 5,56 * 10$^{-5}$ m/s ; $\delta$ = 200 μm.

Das Beispiel zeigt, daß beim Rotationsmodul aufgrund der gleichmäßigen Druckverteilung ein gleichmäßiger Fluß durch das Filtermittel erzeugt wird, mit einer entsprechend engen Verweilzeitverteilung. Damit ist gewährleistet, daß der größte Teil der zur Verfügung stehenden Filtermatrix genutzt werden kann. Dies ist insbesondere dann wichtig, wenn die Filtermatrix aktiv zur Anreicherung eines Gelöststoffes genutzt wird. Im Gegensatz dazu zeigt das Beispiel, daß bei einem konventionellen Tangentialflußfilter, wie dem Hohlfasermodul, eine breite Verweilzeitverteilung aufgrund des longitudalen Druckverlusts auftritt, die z.B. im chromatografieanalogen Betrieb zu einem verfrühten Durchbruch führt, mit einer entsprechend verringerten Kapazitätsausnutzung der Filtermatrix; legt man einen typischen Durchsbruchsgrenzwert von $c/c_o$ = 10% zugrunde, ergibt sich hier eine nur 52%ige Kapazitätsnutzung der Matrix gegenüber >98% beim Rotationsfilter. Neben der verbesserten Kapazitätsausnutzung ist - bedingt durch die engere Verweilzeitverteilung - auch eine verbesserte Konzentrierung bei der Elution des gebundenen Produkts bei Verwendung des Rotationsfilters zu erwarten.

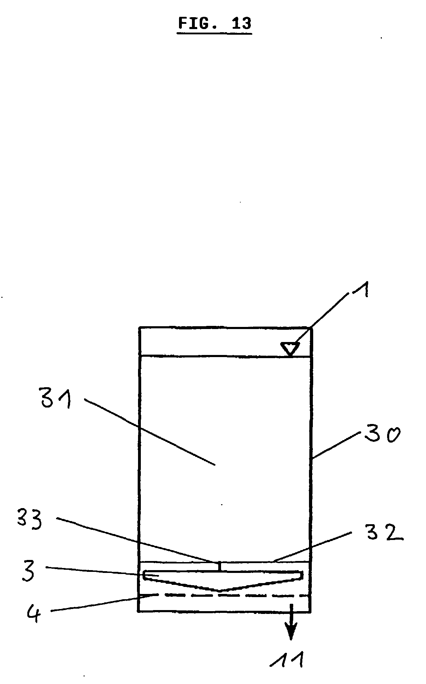

Fig. 13 zeigt eine Rührzelle mit einem konischen Rotor 3 zur absatzweisen Filtration. Die Rührzelle umfaßt ein zylindrisches Gehäuse 30 mit einem konischen Rotor 3, der in dem Gehäuse 30 über dem planaren Filtermittel 4 angeordnet ist. Die Zuführung 1 der zu klärenden Suspension erfolgt in den freien Raum 31 der Rührzelle 30 oberhalb des Rotors 3. In der Rührzelle 30 ist eine Halterung 32 zur Halterung der Achse 33 des Rotors 3 angeordnet. Das Filtrat 11 wird in axialer Richtung der Rührzelle 30 entnommen, während das Retentat in den Raum 31 oberhalb des Rotors 3 zurückfließt. Sowohl die Vorrichtung gemäß der Fig. 2 als auch die Vorrichtung gemäß der Fig. 3 (Doppelmembranrührzelle und Rührzelle mit konischem Rotor) können mittels eines einfachen Labormagnetrührers angetrieben werden, wobei im Rotor Magnetkerne eingelassen sind.

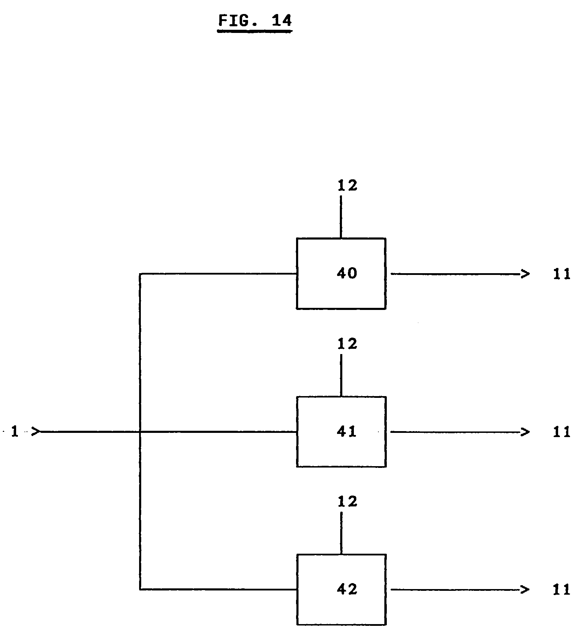

Fig. 14 zeigt die Parallelschaltung mehrerer, hier dreier, Filtervorrichtungen 40, 41 und 42, wobei jede Filtervorrichtung 40, 41 und 42 mit dem Zuführfluß 1 der zu filtrierenden Lösung oder Suspension eingangsseitig versorgt wird. Den Filtervorrichtungen 40, 41, 42 wird parallel das Filtrat 11 sowie das Retentat 12 entnommen. Durch eine derartige Parallelschaltung wird vorteilhafterweise die Gesamtmembranoberfläche des Systems vergrößert, wodurch eine höhere Filtrationsleistung erreicht wird. Durch eine entsprechende Anordnung der Filtrationsvorrichtungen 40, 41, 42 in einer Art Multistackbauweise kann weiterhin beispielsweise erreicht werden, daß die Rotoren (nicht dargestellt) der Filtrationsvorrichtung 40, 41 und 42 von einem gemeinsamen Antrieb angetrieben werden. Weiterhin ist es möglich, die parallelgeschalteten Filtrationsvorrichtung beispielsweise in einem gemeinsamen Gehäuse anzuordnen, um mittels einer modularen Bauweise die Konstruktionskosten zu senken.

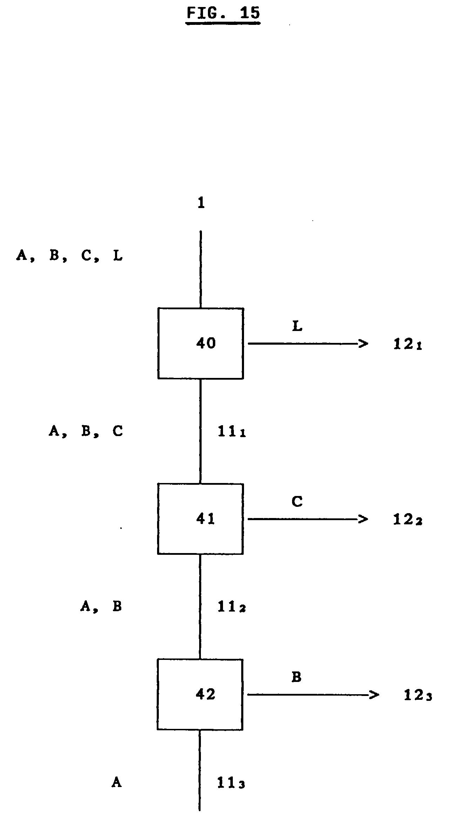

Fig. 15 zeigt eine serielle Anordnung mehrerer Filtrationsvorrichtungen 40, 41, 42, wobei der Zuführfluß 1 der ersten Filtrationsvorrichtung 40 zugeführt wird. Das sich ergebende Filtrat $11_1$ der ersten Stufe wird als Zuführfluß zur zweiten Filtrationsvorrichtung 41 verwendet. Entsprechend wird das Filtrat $11_2$ der zweiten Stufe der Filtrationsvorrichtung 41 als Eingang zur dritten Filtrationsvorrichtung 42 benutzt, die ein Filtrat $11_3$ der dritten Stufe abgibt. Die entsprechenden Filtrationsvorrichtungen 40, 41, 42 geben jeweils ein Retentat $12_1$, $12_2$ und $12_3$ ab. Derartige serielle Anordnungen mehrerer Filtrationsvorrichtungen sind beispielsweise dann sinnvoll, wenn jede Filtrationsvorrichtung 40, 41, 42 mit einem speziellen, von den anderen verschiedenen Filtermittel ausgestattet ist. Demzufolge, wenn beispielsweise der Zuführfluß die Komponenten A, B, C und Reststoffe L enthält, kann eine stufenweise Filtrierung dergestalt durchgeführt werden, daß das Filtrat $11_1$ der ersten Stufe geklärt ist und die Komponenten A, B und C enthält, das Filtrat $11_2$ der zweiten Stufe nur noch Komponenten A, B und das Filtrat $11_3$ der dritten Stufe nur noch Komponenten A enthält. Entsprechend enthält dann das Retentat $12_3$ der dritten Stufe nur noch Komponenten B und das Retentat $12_2$ der zweiten Stufe demzufolge nur noch die Komponenten C, während das Retentat der ersten Stufe die Reststoffe L enthält. Infolgedessen kann das dreistufige Beispiel eine vollständige Trennung des Gemischs A, B, C und L in reine Komponenten A, B und C erreichen.

**Bezugszeichenliste**

| | |
|---|---|
| 1 | - Zuführfluß |
| 2 | - Spalt |
| 3 | - Rotor |
| 4 | - Filtermittel |
| 5 | - Körper |
| 6 | - Konusabschnitt |
| 7 | - Spitze |
| 8 | - Symmetrieachse |
| 9 | - Filterfläche |
| 10 | - Durchflußrichtung |
| 11 | - Filtrat |
| 11o | - Filtrat |
| 11u | - Filtrat |
| $11_1$ | - Filtrat |
| $11_2$ | - Filtrat |
| $11_3$ | - Filtrat |
| 12 | - Retentat |
| 12o | - Retentat |
| 12u | - Retentat |
| $12_1$ | - Retentat |
| $12_2$ | - Retentat |
| $12_3$ | - Retentat |
| 13 | Gehäuse |
| 14 | oberer Konusabschnitt |
| 15 | unterer Konusabschnitt |
| 16 | Filtermittel |
| 17 | Filtermittel |
| 18 | Deckel oben |
| 19 | Deckel unten |
| 20 | Filtratraum oben |
| 21 | Filtratraum unten |
| 22 | Stutzen oben |
| 23 | Stutzen unten |
| 24 | Deckel oben |
| 25 | Deckel unten |
| 26 | Lager oben |
| 27 | Lager unten |
| 28 | Achse |
| 30 | Gehäuse Rührzelle |
| 31 | Rührzellenraum |
| 32 | Halterung |
| 40 | - Filtrationsvorrichtung |
| 41 | - Filtrationsvorrichtung |
| 42 | - Filtrationsvorrichtung |
| s | - maximale Spaltbreite |

$s_i$      - Abstand Konusspitze - Filterfläche
r      - Radius
V      - Vitalität
F      - Drucknormierter Flux
A      - Komponenten
B      - Komponenten
C      - Komponenten
L      - Reststoffe
m      - Affinitätsmembran
t      - Trennschicht/-Membran
a      - aktive Schicht/Membran
P      - Packung
n      - Anzahl

**Patentansprüche**

1. Filtrationsvorrichtung zur Abtrennung und/oder Anreicherung von Gelöststoffen aus einer Suspension mit mindestens einem durchströmten Filtermittel (4), **dadurch gekennzeichnet,** daß auf der Retentatseite des Filtermittels (4) ein Mittel (3) zur Erzeugung eines Scherfeldes angeordnet ist, das ein annähernd homogenes Scherfeld und Druckgefälle über den Einströmquerschnitt des Filtermittels (4) erzeugt.

2. Filtrationsvorrichtung nach Anspruch 1, **dadurch gekennzeichnet**, daß das Mittel (3) zur Erzeugung eines Scherfeldes durch einen rotationssymmetrischen Rotor (3) gebildet wird.

3. Filtrationsvorrichtung nach Anspruch 2, **dadurch gekennzeichnet**, daß der Rotor (3) einen radial nach außen zunehmenden Abstand zum Filtermittel (4) aufweist.

4. Filtrationsvorrichtung nach Anspruch 3, **dadurch gekennzeichnet,** daß der rotationssymmetrische Rotor durch einen konischen Rotor (3) gebildet wird, dessen Konusabschnitt (6, 14, 15) in Richtung zum Filtermittel (4) angeordnet ist.

5. Filtrationsvorrichtung nach Anspruch 4, **dadurch gekennzeichnet,** daß der Konusabschnitt (14, 15) als Kegelstumpf ausgebildet ist.

6. Vorrichtung nach einem der Ansprüche 1 - 5, **dadurch gekennzeichnet,** daß das Filtermittel (4) eine planare Filterfläche (9) aufweist.

7. Vorrichtung nach Anspruch 6, **dadurch gekennzeichnet**, daß das Verhältnis der maximalen Spaltweite s zwischen Filterfläche (9) und Rotor (3) zu Rotorradius r kleiner als 0,2 ist.

8. Vorrichtung nach einem der vorangegangenen Ansprüche, **dadurch gekennzeichnet**, daß der Konuswinkel ($\phi$) des Konusabschnitts (6) im Bereich von < 12°, vorzugsweise bei 3 - 4° liegt.

9. Vorrichtung nach Anspruch 8, **dadurch gekennzeichnet**, daß der Konuswinkel ($\phi$) des Rotors (3) ca. 4° beträgt.

10. Vorrichtung nach Anspruch 9, **dadurch gekennzeichnet**, daß der Abstand Kegelspitze Rotor (3) zur Filterfläche (9) < 5mm.

11. Vorrichtung nach Anspruch 10, **dadurch gekennzeichnet**, daß der Rotordurchmesser des Rotors (3) 70 - 150 mm beträgt.

12. Vorrichtung nach einem der vorangegangenen Ansprüche, **dadurch gekennzeichnet,** daß die Vorrichtung als Querstromfilter betrieben wird.

13. Vorrichtung nach einem der Ansprüche 1 - 11, **dadurch gekennzeichnet**, daß die Vorrichtung mit axialem Retentatauslaß (12) betrieben wird.

14. Vorrichtung nach Anspruch 12, **dadurch gekennzeichnet**, daß der konische Rotor (3) eine axiale Bohrung zur Abführung des Retentats aufweist.

15. Vorrichtung nach Anspruch 14, **dadurch gekennzeichnet**, daß der konische Rotor (3) hohl ist und dessen Oberfläche als Filter ausgebildet ist.

16. Vorrichtung nach einem der vorangegangenen Ansprüche, **dadurch gekennzeichnet**, daß das Filtermittel (4) durch Matrizes gebildet wird, die zur Adsorption der abzutrennenden Gelöststoffe geeignet sind.

17. Vorrichtung nach Anspruch 16, **dadurch gekennzeichnet**, daß das Filtermittel (4) eine oberflächenmodifizierte Membran (a) ist, insbesondere Affinitätsmembranen auf Basis von Mikrofiltrationsmembranen.

18. Vorrichtung nach Anspruch 16 oder 17, **dadurch gekennzeichnet**, daß das Filtermittel (4) aus Stapeln von Membranen oder Schichten (n) besteht.

19. Vorrichtung nach einem der Ansprüche 16-18, **dadurch gekennzeichnet**, daß das Filtermittel (4) aus mehrlagigen Schichten besteht, die unterschiedliche Funktionalitäten haben, insbesondere eine Kombination einer Filterschicht mit einer Adsoptionsschicht oder-packung.

20. Vorrichtung nach einem der vorangegangenen Ansprüche, **dadurch gekennzeichnet**, daß Filtermittel (4) und Rotationsmittel (3) mehrstufig nach dem Rotor-Stator Prinzip angeordnet sind.

21. Vorrichtung nach einem der vorangegangenen Ansprüche, **dadurch gekennzeichnet**, daß das Filtermittel (4) als Rotor (3) ausgelegt ist.

22. Vorrichtung nach einem der vorangegangenen Ansprüche, **dadurch geklennzeichnet**, daß die Vorrichtung eine Chromatografiesäule mit Packung (P) auf der Filtratseite aufweist.

23. Verwendung der Vorrichtung nach einem der Ansprüche 1 bis 21, **dadurch gekennzeichnet**, daß die Vorrichtung zur Querstromfiltration insbesondere von biologischen Suspensionen eingesetzt wird.

24. Verwendung der Vorrichtung nach Anspruch 22, **dadurch gekennzeichnet**, daß die Chromatografiesäule zur Zellabtrennung oder Zellrückführung eingesetzt wird, insbesondere bei kontinuierlicher Perfusionskultur von tierischen Zellen.

25. Verwendung der Vorrichtung nach einem der Ansprüche 1 - 22, **dadurch gekennzeichnet**, daß die Vorrichtung zur integrierten Abtrennung, Anreicherung, Konzentrierung und Reinigung von Gelöststoffen aus partikelhaltigen Suspensionen eingesetzt wird, insbesondere von biologisch aktiven Substanzen aus Zellsuspensionen.

26. Verwendung der Vorrichtung nach einem der Ansprüche 1 - 22, **dadurch gekennzeichnet**, daß die Vorrichtung zur Abtrennung, Anreicherung, Konzentrierung und Reinigung von Gelöststoffen aus partikelhaltigen Suspensionen im chromatografieanalogen Betrieb eingesetzt wird, insbesondere von biologisch aktiven Proteinen.

27. Filtrationssystem unter Verwendung einer Vorrichtung nach einem der Ansprüche 1 bis 22, **dadurch gekennzeichnet**, daß die Filtrationsvorrichtungen (40, 41, 42) hintereinandergeschaltet sind.

28. Filtrationssystem unter Verwendung einer Vorrichtung nach einem der Ansprüche 1 - 22, **dadurch gekennzeichnet**, daß mehrere Filtrationsvorrichtungen parallel (40, 41, 42) geschaltet sind.

**FIG. 1**

a)

b)

c)

FIG. 2

## FIG. 3

FIG. 4

**FIG. 5A**

**FIG. 5B**

FIG. 6

$v = \omega * r$

$\vec{v}_{tangential}(z)$

EP 0 815 927 A2

FIG. 7

$v_{lift}$

$\Omega$

$v_{filtrate}$

$\delta$

3

EP 0 815 927 A2

FIG. 8

## FIG. 9

## FIG. 10

Druck [mbar]

rel. Länge od. Radius [-]

EP 0 815 927 A2

Flux [L/hm^2]

rel. Länge od. Radius [-]

EP 0 815 927 A2

FIG. 12

## FIG. 13

## FIG. 14

## FIG. 15

1

A, B, C, L

```
          ┌────────┐      L
          │   40   │  ─────────────>   12₁
          └────────┘
```

A, B, C        11₁

```
          ┌────────┐      C
          │   41   │  ─────────────>   12₂
          └────────┘
```

A, B          11₂

```
          ┌────────┐      B
          │   42   │  ─────────────>   12₃
          └────────┘
```

A          11₃